# EUROPEAN PATENT SPECIFICATION

(11) **EP 0 537 260 B1**
(45) Date of publication and mention of the grant of the patent: **29.03.1995**
(21) Application number: 91912791.0
(22) Date of filing: 13.06.1991
(51) Int. Cl.: C07C 43/11, C07C 41/03

(54) **A METHOD FOR AVOIDING FORMATION OF BY-PRODUCTS AT THE PRODUCTION OF AN ETHOXYLATED POLYOL**
VERFAHREN ZUR VERMEIDUNG DER BILDUNG VON NEBENPRODUKTEN BEI DER HERSTELLUNG VON ETHOXYLIERTEN POLYOLEN
PROCEDE SERVANT A EVITER LA FORMATION DE SOUS-PRODUITS DURANT LA FABRICATION D'UN POLYOL ETHOXYLE

(30) Priority: 02.07.1990 SE 9002316
(43) Date of publication of application: 21.04.1993
(73) Proprietor: PERSTORP AB, 284 80 Perstorp (SE)
(72) Inventor: BERGVALL, Göran, S-284 00 Perstorp (SE)
(74) Representative: Hansen, Bernd, Dr. Dipl.-Chem.
(86) International application number: SE9100430
(87) International publication number: WO9200265

(56) References cited:
- EP-A- 0 141 253
- DE-A- 3 431 156
- GB-A- 1 480 432
- US-A- 3 190 927
- US-A- 4 767 846

## Description

The present invention relates to a method for avoiding formation of disturbing amounts of by-products in the form of monoethylene glycol, diethylene glycol and triethylene glycol at the production of an ethoxylated polyol of low molecular weight.

Polyols such as monopentaerythritol, dipentaerythritol, tripentaerythritol, trimethylolethane and neopentyl glycol are essential raw materials within a number of different chemical fields. Polyols play an important role as a raw material at the production of radiation curing acrylates for instance to be used in the coatings industry. Then ethoxylated polyols are used more and more since they combine good technical and hygienic properties.

At a normal ethoxylation of polyols which are not in a liquid state at the reaction temperature, the reaction is carried out with water and/or other polar solvents present to get the reaction to take place in a one phase system. At the process by-products, for instance monoethylene glycol, diethylene glycol and/or triethylene glycol are formed. They are very difficult to remove since they have a low volatility, form azeotropic mixtures and have a strong affinity to the main product. In practice it is very difficult to get below 0.4% by weight of diethylene glycol.

At the production of acrylate monomers the presence of ethylene glycols in the ethoxylated polyol results in a formation of corresponding acrylates of monoethylene glycol, diethylene glycol and triethylene glycol. These acrylates are toxic and irritating to the skin. This is valid especially for diethylene glycol diacrylate.

According to the present invention a wholly new method for avoiding formation of disturbing amounts of monoethylene glycol, diethylene glycol and triethylene glycol at the production of an ethoxylated polyol of low molecular weight has been brought about.

The method comprises first reacting a polyol with propylene oxide in the presence of water or another polar solvent at a molar ratio of 1:0.5 - 1:2.5, preferably 1:0.6 - 1:1.5 and in the presence of an alkali metal hydroxide catalyst. Solvent as well as propylene glycols and ethers formed are then evaporated.

The polyol propoxylate obtained is then reacted with ethylene oxide at a molar ratio of 1:2 - 1:10 in the presence of an alkali metal hydroxide catalyst, whereby an ethoxylated polyol propoxylate with an average molecular weight of 270-500, preferably 300-400 is obtained.

Unreacted ethylene oxide and ethers formed are usually evaporated from the reaction mixture. Suitably the ethoxylated polyol propoxylate is neutralized to pH 6-8 by means of acid sodium pyrophosphate, whereby a product with a low content of ashes is obtained.

Preferably the polyol consists of monopentaerythritol but also dipentaerythritol, tripentaerythritol, trimethylolethane or neopentyl glycol can be used.

The alkali metal hydroxide catalyst is suitably present in solid state. Usually potassium hydroxide is preferred as a catalyst but also other alkali metal hydroxides can be used. Solutions of alkali metal hydroxide catalysts may also be used.

As a final product a clear almost colourless liquid consisting of ethoxylated polyol propoxylate mainly containing propylene glycols as impurities is obtained. The content of each of monoethylene glycol, diethylene glycol and triethylene glycol is less than 0.2% by weight, preferably less than 0.1% by weight. Propylene glycols are easier to evaporate than corresponding ethylene glycols. Moreover, propylene glycol acrylates have a very low toxicity.

The invention is disclosed further in connection with the embodiment examples below, of which example 1 illustrates the invention and examples 2 and 3 show comparison tests. Example 2 shows the importance of the propoxylation for obtaining a low content of impurities, especially diethylene glycols and triethylene glycols. Example 3 illustrates the difficulty to remove these impurities from the product.

### Example 1

136 g (= 1 mole) pentaerythritol, 3.6 g powdered KOH and 70 g distilled water were charged into a 1-litre laboratory autoclave. The mixture was heated to 150^{o}C at stirring and nitrogen gas atmosphere.

Then 58 g propylene oxide (= 1 mole) was pumped in for 1 hour at 150^{o}C and at a pressure of 2000-4000 mm Hg. A postreaction was carried out for 30 minutes at 150^{o}C.

Thereafter water was evaporated at 150^{o}C and <1 mm Hg until a water content of <0.2% was obtained in the autoklave.

162,8 g ethylene oxide (= 3.7 moles) was then charged for 2 hours at 150^{o}C and a pressure of 2500-4000 mm Hg (nitrogen gas atmosphere).

A subreaction took place for 30 minutes at 150^{o}C. Any possibly unreacted ethylene oxide was evaporated at 150^{o}C and <1 mm Hg.

The product was neutralized with 3% acid sodium pyrophosphate together with 1.5% water and 1.5% Celite (filter aid) to pH 6-8. After stirring for 1 hour at 130^{o}C water was evaporated at 130^{o}C and <1 mm Hg for 30 minutes. The product was then filtered at 100^{o}C.

Finally the product was stabilized with 250 ppm butylhydroxy toluene, BHT. 339 g ethoxylated pentaerythritol propoxylate in the form of a low viscous clear liquid with an average molecular weight of 356 and an OH-number of 629 mg KOH was obtained as a final product. The yield amounted to 95%. At an analysis of the product by means of gas chromatography the content of monoethylene glycol was determined to 0.03%, diethylene glycol to 0.05 % and triethylene glycol to 0.06%. The content of Na⁺ and K⁺ was < 100 ppm.

### Example 2

136 g (= 1 mole) pentaerythritol, 3.6 g powdered KOH and 70 g distilled water were charged into a 1-litre laboratory autoclave. The mixture was heated to 150^{o}C at stirring and nitrogen gas atmosphere.

Then 57.2 g ethylene oxid (= 1.3 moles) was pumped in for 1 hour at 150^{o}C and at a pressure of 2000-4000 mm Hg. A postreaction was carried out for 30 minutes at 150^{o}C.

Thereafter water was evaporated at 150^{o}C and <1 mm Hg until a water content of <0.2% was obtained in the autoclave.

162.8 g ethylene oxide (= 3.7 moles) was then charged for 2 hours at 150^{o}C and a pressure of 2500-4000 mm Hg (nitrogen gas atmosphere).

A subreaction took place for 30 minutes at 150^{o}C. Any possibly unreacted ethylene oxide was evaporated at 150^{o}C and <1 mm Hg.

The product was neutralized with 3% acid sodium pyrophosphate together with 1.5% water and 1.5% Celite (filter aid) to pH 6-8. After stirring for 1 hour at 130^{o}C water was evaporated at 130^{o}C and <1 mm Hg for 30 minutes. The product was then filtered at 100^{o}C.

Finally the product was stabilized with 250 ppm butylhydroxy toluene, BHT. 338.2 g ethoxylated pentaerythritol in the form of a low viscous clear liquid with an average molecular weight of 356 and an OH-number of 630 mg KOH was obtained as a final product. The yield amounted to 95%. At an analysis of the product by means of gas chromatography the content of monoethylene glycol was determined to 0.1%, diethylene glycol to 0.9% and triethylene glycol to 2.6%. The content of Na⁺ and K⁺ was <100 ppm.

### Example 3

Water, ethanol and xylene respectively was added to the final product obtained in Example 2 in an effort to decrease the content of monoethylene glycol, diethylene glycol and triethylene glycol at a subsequent evaporation at vacuum. Then 5% solvent was added in two steps and an evaporation was carried out for 2 hours at 125^{o}C and 0.1 mm Hg.

At an analysis the contents of monoethylene glycol, diethylene glycol and triethylene glycol were determined to 0.02%, 0.25% and 2.1% respectively with water as a solvent. With ethanol as a solvent the contents were 0.02%, 0.23% and 1.5% respectively and with xylene as a solvent the contents were 0.02%, 0.29% and 1.8% respectively.

## Claims

1. A method for avoiding formation of disturbing amounts of by-products in the form of monoethylene glycol, diethylene glycol and triethylene glycol at the production of an ethoxylated polyol of low molecular weight, which comprises first reacting a polyol with propylene oxide in the presence of water or another polar solvent at a molar ratio of 1:0.5 - 1:2.5, preferably 1:0.6 - 1:1.5 and in the presence of an alkali metal hydroxide catalyst, evaporating solvent as well as propylene glycols and ethers formed, reacting the polyol propoxylate obtained with ethylene oxide at a molar ratio of 1:2 - 1:10 in the presence of an alkali metal hydroxide catalyst whereby an ethoxylated polyolpropoxylate with an average molecular weight of 270-500, preferably 300-400 is obtained.

2. A method according to claim 1, wherein the polyol preferably consists of monopentaerythritol.

3. A method according to claim 1 or 2, wherein unreacted ethylene oxide and ethers formed are evaporated from the reaction mixture.

4. A method according to anyone of claims 1-3, wherein the ethoxylated polyol propoxylate is neutralized to pH 6-8 by means of acid sodium pyrophosphate, whereby a product with a low content of ashes is obtained.

5. A method according to anyone of claims 1-4, wherein potassium hydroxide is used as a catalyst.

6. A method according to anyone of claims 1-5, wherein the alkali metal hydroxide catalyst is present in solid state.

7. A method according to anyone of claims 1-6, wherein the content of each of monoethylene glycol, diethylene glycol and triethylene glycol in the final product is less than 0.2% by weight, preferably less than 0.1% weight.

## Patentansprüche

1. Verfahren zum Vermeiden der Bildung von störenden Mengen von Nebenprodukten in Form von Monoethylenglykol, Diethylenglykol und Triethylenglykol bei der Herstellung eines ethoxylierten Polyols mit niedrigem Molekulargewicht, umfassend das erste Umsetzen eines Polyols mit Propylenoxid in Gegenwart von Wasser oder einem anderen polaren Lösungsmittel, in einem Mol-Verhältnis von 1:0,5 bis 1:2,5, vorzugsweise 1:0,6 bis 1:1,5, und in Gegenwart eines Alkalimetallhydroxidkatalysators, das Verdampfen des Lösungsmittels sowie von gebildeten Propylenglykolen und Ethern, Umsetzen des erhaltenen Polyolpropoxylats mit Ethylenoxid in einem Mol-Verhältnis von 1:2 bis 1:10 in Gegenwart eines Alkalimetallhydroxidkatalysators unter Erhalt eines ethoxylierten Polyolpropoxylats mit einem Durchschnittsmolekulargewicht von 270 bis 500, vorzugsweise 300 bis 400.

2. Verfahren gemäß Anspruch 1, bei dem das Polyol vorzugsweise aus Monopentaerythrit besteht.

3. Verfahren gemäß Anspruch 1 oder 2, bei dem das nichtumgesetzte Ethylenoxid und gebildete Ether aus dem Reaktionsgemisch abgedampft werden.

4. Verfahren gemäß einem der Ansprüche 1 bis 3, bei dem das ethoxylierte Polyolpropoxylat auf den pH-Wert 6 bis 8 neutralisiert wird mittels saurem Natriumpyrophosphat, wobei man ein Produkt mit einem niedrigen Aschegehalt erhält.

5. Verfahren gemäß einem der Ansprüche 1 bis 4, bei dem Kaliumhydroxid als Katalysator verwendet wird.

6. Verfahren gemäß einem der Ansprüche 1 bis 5, bei dem der Alkalimetallhydroxidkatalysator im festen Zustand vorliegt.

7. Verfahren gemäß einem der Ansprüche 1 bis 6, bei dem der Gehalt an jeweils Monoethylenglykol, Diethylenglykol und Triethylenglykol in dem Endprodukt weniger als 0,2 Gew.-%, vorzugsweise weniger als 0,1 Gew.-% beträgt.

## Revendications

1. Procédé pour éviter la formation de quantités indésirables de sous-produits sous forme de monoéthylèneglycol, de diéthylèneglycol et de triéthylèneglycol lors de la production d'un polyol éthoxylé de faible masse moléculaire, consistant dans un premier temps à faire réagir un polyol avec de l'oxyde de propylène en présence d'eau ou d'un autre solvant polaire avec un rapport molaire compris entre 1:0,5 et 1:2,5, de préférence entre 1:0,6 et 1:1,5 en présence d'un catalyseur de type hydroxyde de métal alcalin, à évaporer le solvant ainsi que les propylèneglycols et éthers formés, à faire réagir le polyol propoxylé obtenu avec de l'oxyde d'éthylène avec un rapport molaire compris entre 1:2 et 1:10 en présence d'un catalyseur de type hydroxyde de métal alcalin ce qui permet d'obtenir un polyol propoxylé et éthoxylé ayant une masse moléculaire moyenne comprise entre 270 et 500, de préférence entre 300 et 400.

2. Procédé conforme à la revendication 1 dans lequel le polyol est de préférence le monopentaérythritol.

3. Procédé conforme à la revendication 1 ou 2 dans lequel l'oxyde d'éthylène n'ayant pas réagi et les éthers formés sont éliminés du mélange réactionnel par évaporation.

4. Procédé conforme à une quelconque des revendications 1 à 3 dans lequel le polyol propoxylé et éthoxylé est neutralisé jusqu'à pH 6 - 8 avec du pyrophosphate acide de sodium, ce qui permet d'obtenir un produit ayant une faible teneur en cendres.

5. Procédé conforme à une quelconque des revendications 1 à 4 dans lequel on utilisé comme catalyseur de l'hydroxyde de potassium.

6. Procédé conforme à une quelconque des revendications 1 à 5 dans lequel le catalyseur de type hydroxyde de métal alcalin est présent sous forme d'un solide.

7. Procédé conforme à une quelconque des revendications 1 à 6 dans lequel la teneur en monoéthylèneglycol, en diéthylèneglycol et en triéthylèneglycol dans le produit final est pour chacun de ces composés inférieure à 0,2 %, de préférence inférieure à 0,1 % en poids.
